# EUROPEAN PATENT APPLICATION

(11) **EP 2 364 638 A1**
(43) Date of publication of application: **14.09.2011**
(21) Application number: 10156379.9
(22) Date of filing: 12.03.2010
(51) Int. Cl.: A61B 5/00

(54) **A care monitoring system**

(71) Applicant: Valentia Projects Limited, Dublin 18 (IE)
(72) Inventor: Javad, Ahmed, 18, Dublin (IE); Nelson, Peter, 18, Dublin (IE); Nolan, Yvonne, 18, Dublin (IE); Ali, Mubashir Raza, 18, Dublin (IE)
(74) Representative: Roche, Dermot

(57) **Abstract**

A computer implemented care monitoring system (1) remotely monitors a person requiring care in their home. The system (1) comprises a monitoring hub (2), a plurality of monitoring devices (3), and a server (4). The monitoring hub (2) may be connected in communication with the plurality of monitoring devices (3) to receive alarm data and/or non-alarm data from the plurality of monitoring devices (3). The monitoring hub (2) comprises a user interface to receive alarm data and/or non-alarm data from a user. The monitoring hub (2) determines whether the data received is alarm data or non-alarm data. If the data received is determined to be alarm data, the monitoring hub (2) transfers the data received to the server (4). The monitoring hub (2) determines whether the monitoring hub (2) is connected in communication with the server (4), and the user interface indicates to the user whether the monitoring hub (2) is connected in communication with the server (4).

## Description

### Introduction

This invention relates to a care monitoring system.

### Statements of Invention

According to the invention there is provided a care monitoring system comprising means to receive alarm data and/or non-alarm data from one or more sources, means to determine whether the data received is alarm data, and responsive to the data received being determined to be alarm data, means to transfer the data received to a remote location.

In one embodiment of the invention the means to receive data is connectable in communication with one or more monitoring devices to receive alarm data and/or non-alarm data from the one or more monitoring devices. Each monitoring device may automatically measure a variety of readings related to the user or the user's surroundings. Preferably the means to receive data is connectable in communication with a first monitoring device by means of a first communication protocol, and the means to receive data is connectable in communication with a second monitoring device by means of a second communication protocol, the first communication protocol being different from the second communication protocol. In this manner the system of the invention may be used with a variety of different monitoring devices with a variety of different communication protocols from a variety of different device manufacturers. Ideally the means to receive data is configured to establish communication with a monitoring device by transmitting a plurality of communication protocol signals to the monitoring device until communication is established. In this manner the means to receive data is capable of establishing communication with a variety of different monitoring devices with a variety of different communication protocols from a variety of different device manufacturers.

In another embodiment the means to receive data comprises a user interface to receive alarm data and/or non-alarm data from a user.

In one case responsive to the data received being determined to be non-alarm data, the system comprises means to store the data received locally.

In another case the system comprises means to determine whether the means to transfer the data is connected in communication with a remote location. Preferably the system comprises means to indicate to a user whether the means to transfer the data is connected in communication with a remote location. In this manner the system of the invention provides peace of mind to a user to be certain that the system is fully operational in the event that the user wishes to transmit alarm data to the remote location. Ideally the system comprises means to determine the latency between the means to transfer the data and a remote location. Most preferably the system comprises means to indicate to a user the latency between the means to transfer the data and a remote location.

The means to transfer the data may be configured to transfer the data by means of a telephone network. The means to transfer the data may be configured to transfer the data by means of an internet connection. By having more than one means to transfer the data, this provides the system with enhanced reliability.

In one embodiment the system comprises one or more monitoring devices connected in communication with the means to receive data.

In another embodiment the system comprises a computer implemented system.

The invention also provides in another aspect a method of care monitoring, the method comprising the steps of
receiving alarm data and/or non-alarm data from one or more sources,
determining whether the data received is alarm data, and
responsive to the data received being determined to be alarm data, transferring the data received to a remote location.

In one embodiment of the invention the alarm data and/or non-alarm data is received from one or more monitoring devices. Each monitoring device may automatically measure a variety of readings related to the user or the user's surroundings. Preferably the method comprises the step of establishing communication with a monitoring device by transmitting a plurality of communication protocol signals to the monitoring device until communication is established. In this manner the invention is capable of establishing communication with a variety of different monitoring devices with a variety of different communication protocols from a variety of different device manufacturers.

In another embodiment the alarm data and/or non-alarm data is received from a user by means of a user interface.

In one case the method comprises the step of, responsive to the data received being determined to be non-alarm data, storing the data locally.

In another case the method comprises the step of determining whether a means to transfer data is connected in communication with a remote location. Preferably the method comprises the step of indicating to a user whether a means to transfer data is connected in communication with a remote location. In this manner the invention provides peace of mind to a user to be certain that the invention is fully operational in the event that the user wishes to transmit alarm data to the remote location. Ideally the method comprises the step of determining the latency between a means to transfer data and a remote location. Most preferably the method comprises the step of indicating to a user the latency between a means to transfer data and a remote location.

In another aspect of the invention there is provided a computer program product comprising computer program code capable of causing a computer system to perform a method of the invention when the computer program product is run on a computer system.

The computer program product may be embodied on a record medium. The computer program product may be embodied on a carrier signal. The computer program product may be embodied on a read-only memory.

### Brief Description of the Drawings

The invention will be more clearly understood from the following description of an embodiment thereof, given by way of example only, with reference to the accompanying drawings, in which:
Fig. 1 is a schematic illustration of a care monitoring system according to the invention,
Fig. 2 is a schematic illustration of the system of Fig. 1 in use,
Fig. 3 is a flowchart illustrating calculation of a quality of life metric,
Fig. 4 is a flowchart illustrating transfer of alarm data,
Fig. 5 is a flowchart illustrating receipt of data from one or more monitoring devices,
Fig. 6 is a schematic illustration of a user interface of the system of Fig. 1,
Fig. 7 is a flowchart illustrating determining whether a means to transfer data is connected in communication with a remote location,
Fig. 8 is a flowchart illustrating operation of a control centre,
Fig. 9 is a flowchart illustrating creating appointments,
Fig. 10 is a flowchart illustrating registering administration machines,
Fig. 11 is a flowchart illustrating registering administration clients,
Fig. 12 is a flowchart illustrating network monitoring,
Fig. 13 is a flowchart illustrating systems monitoring, and
Fig. 14 is a flowchart illustrating system detail summary.

### Detailed Description

In this patent specification the term `client' will be understood to mean a person who is being monitored by the invention.

In this patent specification the term 'caregiver' will be understood to mean a healthcare professional that is given access to the system to view authorised client data. This may be a member of any client's care team such as a hospital consultant, a public health nurse, a general practitioner (GP), a pharmacist.

In this patent specification the term 'control centre' will be understood to mean a monitoring centre which may be operated 24 hours a day and 7 days a week by staff to respond to any events that occur within the home and initiate the appropriate actions such as dispatching an ambulance, calling a family member or making a GP appointment. Staff may be clinical or non-clinical depending on individual requirements. The control centre application allows automatic responses where suitable, without requiring input from a controller.

In this patent specification the term 'controller' will be understood to mean a staff member in the control centre. They will use the control centre application to receive calls or alerts from the client system and to respond to the events.

In this patent specification the term 'device call' will be understood to mean a call from telecare base-stations when a telecare alert is received from a telecare device. In this patent specification the term 'device hubs' will be understood to mean device hubs for telecare units in the homes. These units interact with telecare devices in the home wirelessly over the European Social Alarm Frequency band. When an alert is generated, the device hub will initiate a phone call to a pre-programmed number. Information about the type of alert that has been generated is sent using DTMF tones according to a defined protocol such as BS 8521 2009.

In this patent specification the term `DTMF' will be understood to mean Dual-Tone Multi-Frequency which is used for sending data over analogue telephone lines.

In this patent specification the term 'telecare devices' will be understood to mean devices used for monitoring the home and client's ambient environment such as pendants, door contacts, motion detectors, smoke alarms, bed pressure mats, flood detectors. The telecare devices interact with device hubs wirelessly over the European Social Alarm frequency band whenever an alert occurs.

In this patent specification the term 'telecare alerts' will be understood to mean alerts generated from telecare devices e.g. pendant button is pressed, bed is unoccupied for longer than preset time.

In this patent specification the term 'telehealth' will be understood to mean medical devices within the home such as weighing scales or blood pressure monitors which are integrated with the invention using Bluetooth Continua specification and other protocols.

In this patent specification the term 'panic calls' will be understood to mean all calls received from home user application by pressing a software panic connect button.

In this patent specification the term 'family and friends' will be understood to mean family and friends of a client who are authorised to view data and take part in the care of the client.

In this patent specification the term 'portal' will be understood to mean a secure website requiring authentication which can be used by family, friends and caregivers to review data about the client with appropriate permissions.

In this patent specification the term `PSTN' will be understood to mean Public Service Telephone Network including traditional landline - Plain Old Telephone Service (POTS), and GSM mobile telephony.

In this patent specification the term 'real-time' will be understood to mean data is processed instantly as it is available without requiring storage and processing at a later stage.

In this patent specification the term 'third party' will be understood to mean a third party service provider who may offer services through the invention e.g. taxi companies, home shopping, online shopping.

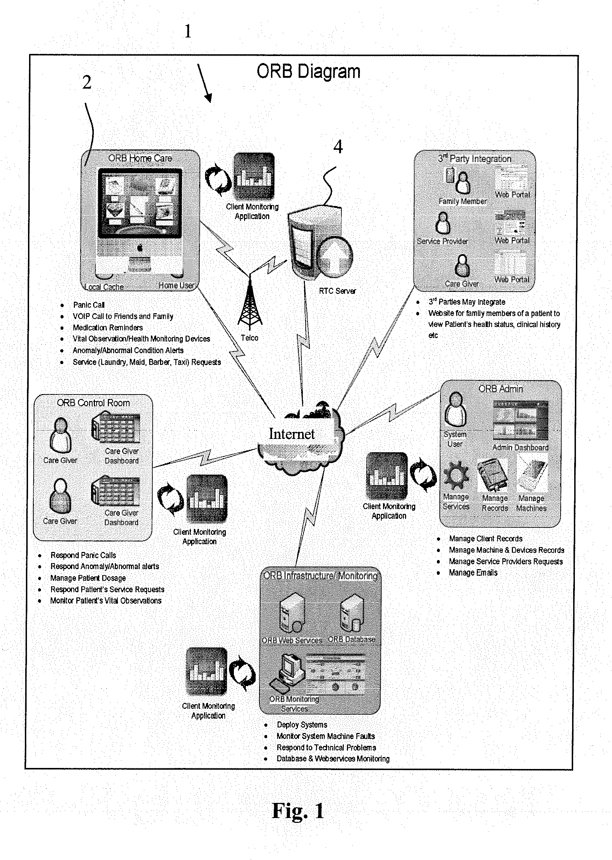

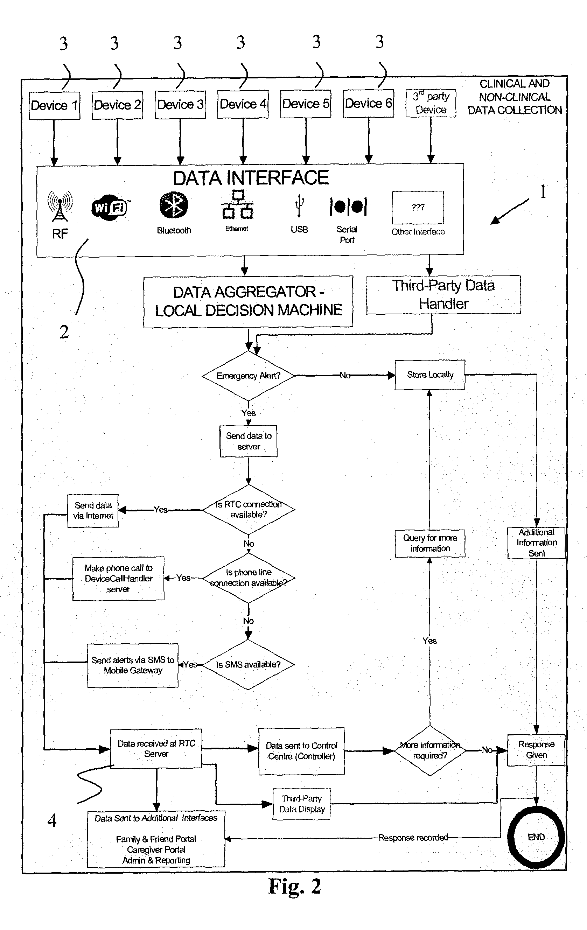

Referring to the drawings, and initially to Figs. 1 and 2 thereof, there is illustrated a computer implemented care monitoring system 1 according to the invention. The system 1 may be employed to remotely monitor a person requiring care in their home, for example an elderly or ill person. The system 1 comprises a monitoring hub 2, a plurality of monitoring devices 3, and a server 4. The monitoring hub 2 and the monitoring devices 3 would typically be located in the person's home. The server 4 would typically be located remotely.

The monitoring hub 2 may be connected in communication with the plurality of monitoring devices 3 to receive alarm data and/or non-alarm data from any of the plurality of monitoring devices 3. Each monitoring device 3 may be a panic button device or a blood pressure measurement device or a room thermometer device or the like. Alarm data may be in the form of a signal from a panic button device or the like. Non-alarm data may be in the form of a signal from a blood pressure measurement device or a room thermometer device or the like. Two or more of the monitoring devices 3 may have different communication protocols. The monitoring hub 2 is able to communicate with each one of the plurality of monitoring devices 3 with the different communication protocols. In particular the monitoring hub 2 establishes communication with each monitoring device 3 by transmitting a plurality of communication protocol signals in series to the particular monitoring device 3 until communication is established with the particular monitoring device 3.

The system 1 is device agnostic. The system 1 may present data obtained from different devices 3 by different manufacturers within one single interface enabling a contextually rich view of data within the client's environment. The device agnostic features of the system 1 include the following components:
the device call handler may receive DTMF tones from different protocols and
interpret the data correctly depending on which protocol is being used;
the invention 1 may integrate data from different types of medical devices 3 into a single platform, to enable contextually rich decisions to be made. The system 1 is not restricted by the capabilities or limitations of any single device manufacturer. This provides a very flexible and demand lead solution which is fully customisable and may be tailored to individual requirements;
any terminal may be used to present the home user application to the client. A terminal may be selected based on individual client's requirements e.g. touch-screen terminal, large screen, web-based browser, mobile application that can be used outside the home;
the control centre application, caregiver portal and family and friend portal may be accessed from multiple locations by multiple people. The data obtained from the client's environment may be presented in multiple locations to multiple people e.g. GP surgery, control centre, hospital consultant, specialist, public health nurse, family and friends.

The invention 1 enables data from devices 3 from multiple device manufacturers to be combined within a single platform. The device call handler application is capable of accepting calls from multiple device hubs 3 using different protocols simultaneously. There may be a number of different social alarm protocols used for assigning DTMF tones to device codes and device locations including BS 8521 2009. When any device hub 3 calls the central server 2, the call will be "answered" by the device call handler application. All the device hubs 3, regardless of which social alarm protocol is being used, require that a data request tone is sent back by the receiving application before the device hub 3 will begin sending information using DTMF tones coded in whichever protocol the device hub 3 is using. However device hubs 3 using different protocols will require a different set of DTMF tones for the "Data Request Tone". The device call handler application will cycle through each of the Data Request Tones for the all supported protocols, using hit and miss until it finds the correct protocol that is required by the device hub 3. Once the device hub 3 begins sending data, the device call handler application will be able to determine what protocol the device hub 3 is using based on differences in the layout of the information received. It may then convert the interpreted DTMF characters into meaningful information such as the device hub ID which identifies the caller, device code and the device location. Medical device manufacturers may easily integrate their monitoring devices 3 within the system 1 and show the collected data through their application on the other end, without requiring modifications to the invention 1.

The monitoring hub 2 comprises a user interface to receive alarm data and/or non-alarm data from a user, for example in the form of a touch screen interface.

The monitoring hub 2 determines whether the data received is alarm data or non-alarm data. If the data received is determined to be alarm data, the monitoring hub 2 transfers the data received to the server 4 at a remote location. The monitoring hub 2 may transfer the data to the server 4 by means of a telephone network for example using a Dual Tone Modem Frequency (DTMF) connection, or by means of an internet connection for example using an Internet Protocol (IP) connection. The data may be further transferred on from the server 4, for example to a database for storage, or to a control centre to enable action to be taken, or to a medical caregiver, or to a family member or friend. If the data received is determined to be non-alarm data, the monitoring hub 2 stores the data received locally at the monitoring hub 2.

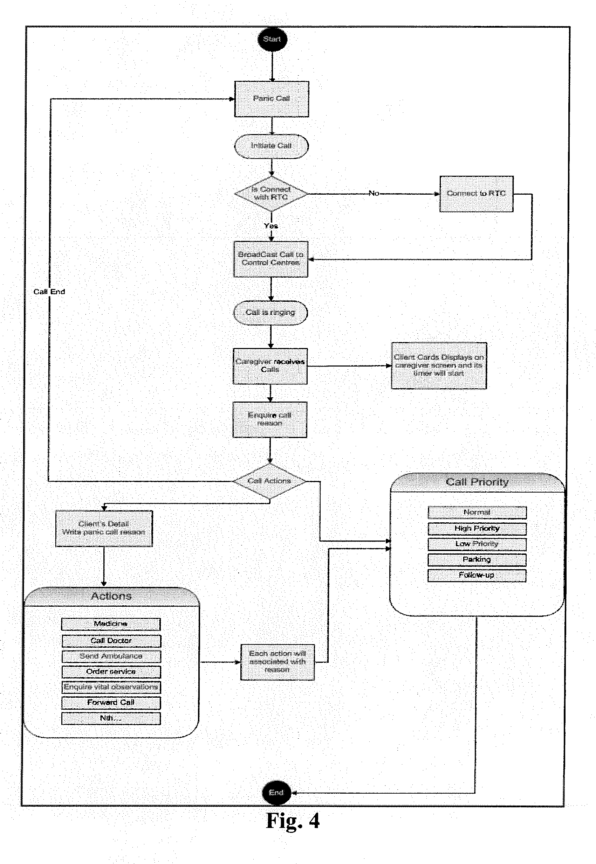

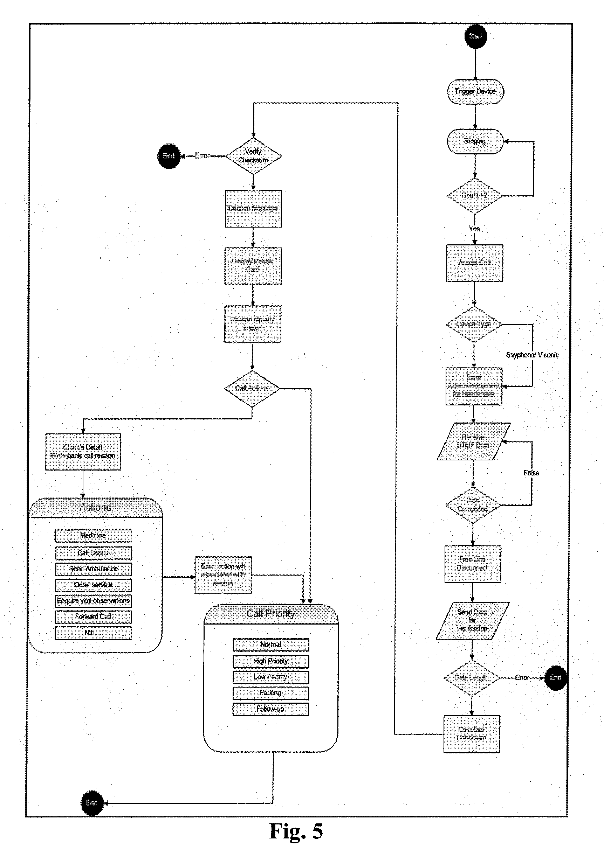

The system 1 is compatible with both panic calls from a home user application panic connect button (Fig. 4) and device calls from a device hub 3 (Fig. 5). The panic connect button calls from the home user application may operate in a number of communications modes:
traditional phone line/"Plain Old Telephone Service" (POTS) initiates phone call to the control centre through traditional phone network on PSTN, or
internet/IP initiates VOIP session through the RTC server 4, or
GSM phone line initiates phone call to the control centre through traditional phone network, or
mobile internet initiates mobile-based VOIP session using technology such as 3G or GPRS through the RTC server 4, or
SMS sends SMS to control centre.

This allows the system 1 to be used in situations where the IP line has failed or where the application is being used from a mobile device outside of the home. Device calls use traditional phone calls which use POTS/PSTN and send information using Dual Tone Multi Frequency (DTMF) tones. The system 1 works in dual mode i.e. PSTN/GSM & TCP/IP. The advantage would be that person would be able to leverage both remote monitoring services which are PSTN based and also services that are IP based. This will work in the event that IP connectivity is lost, all the important data will be communicated via DTMF to the control centre via PSTN or GSM modem. The terminal 2 will support both simultaneously. The control centre software combines DTMF data with IP/Binary data for construction or events timeline.

The monitoring hub 2 determines whether the monitoring hub 2 is connected in communication with the server 4 at the remote location, and determines the latency between the monitoring hub 2 and the server 4 at the remote location. The user interface of the monitoring hub 2 indicates to the user whether the monitoring hub 2 is connected in communication with the server 4 at the remote location, and indicates to the user the latency between the monitoring hub 2 and the server 4 at the remote location. The indication may be by means of a visible and/or audible stimulus at the user interface.

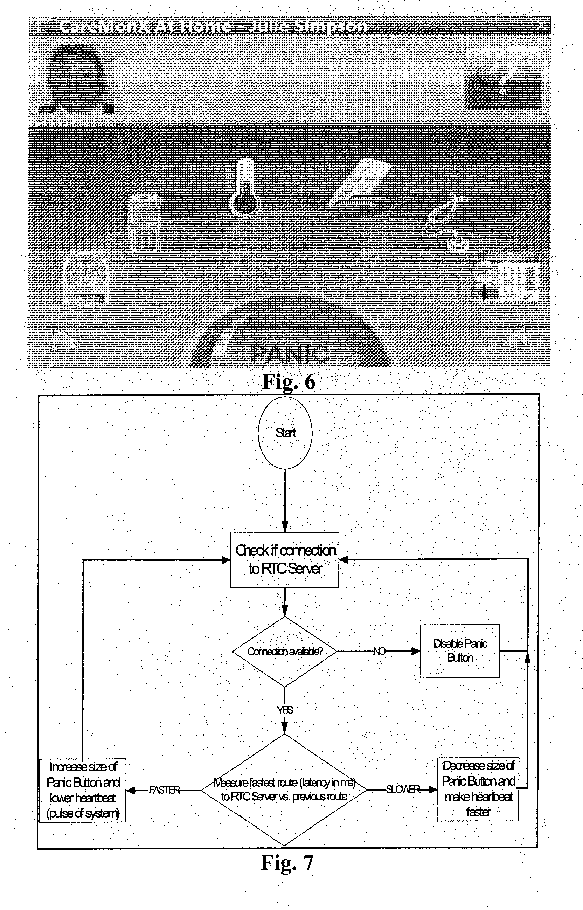

Fig. 4 illustrates the process flow of a panic call from the home user application by means of a software red button panic connect button. This may be installed on a touch-screen in the home or on a mobile device. The home user application has a prominent panic connect button which is visible from all screens (Fig. 6). This panic connect button has the following characteristics:
graphical: the panic connect button may be configured to suit the requirements of the user. In Fig. 6 it is shown as a red semi-circular button;
multilingual: the panic connect button may be translated into different languages;
pre configured rules: when the panic connect button is pressed it will start a panic call. The method used to start the panic call is shown in Fig. 4.

The invention 1 includes a "Connectivity Feedback" feature on the panic connect button. When the button is connected to the RTC server 4 it will be displayed in the normal colour which may be red by default. The system 1 constantly measures the latency between the RTC server 4 and the home user application 2. Where there is a short latency the button size will be increased to indicate a strong connection. Where there is a higher latency the button size will decrease to indicate that the connection is weaker. The purpose of this feature is to ensure that the client is reassured that when the panic connect button is "ON" and pressed, it will reliably start a panic call. When there is no connection to the RTC server 4, the system 1 will attempt to start a telephone call to the control centre application either over the landline/POTS or through the GSM network if running on a mobile phone. If this call cannot be created then the system 1 will send an SMS. If there is no connection the button will be disabled which may be greyed out. Also the panic connect button may pulse according to the strength of the connection which may be in the form of a heartbeat. This feature is shown in Fig. 7. The software based configurable panic button may be customised by the client for size, shape and colour with over lay writing.

Fig. 5 illustrates the process flow of a device call. A device call may come from any device 3 in the home which is configured to send emergency alerts. This may be a wearable pendant panic button, smoke alarm, flood detector or an alert generated from a combination of sensors 3. The call is received into the device call handler application and the DTMF tones are interpreted by the application and passed to the control centre application via the RTC server 4. Depending on the configuration, the RTC server 4 will broadcast the call to all instances of the control centre application or will select a single instance using standard algorithms for selection.

Based on the nature of the generated alerts e.g. panic call, device call or rules generated, it is possible to configure the system 1 to download additional information from the home when an alert occurs. This may be offered by this system 1 since it is monitoring a wide number of events in the home including time since the client last visited the kitchen may be used to infer mealtimes, time that the client spent in bed, motion in different rooms in the home, communications with family members and the like. The event-driven data download will present the controller with a summary of events over a specified number of hours to create a contextually rich information bundle that can be used to enable more efficient decision making, intervention protocol and resource use. For example, if a device call occurs to the controller during the night from the pendant, it may not be immediately obvious if the client is in distress or if they have just accidentally left the pendant on the bed and rolled on top of it in their sleep. The event-driven data download may be used to obtain a large amount of information that may be used by the controller to assist in making a decision, for example:
bed pressure sensor activated - is client in bed?
bedside fall mats - has client fallen getting out of bed?
door sensor on bedroom door - has client left bedroom?
door sensor on bathroom door - has client reached bathroom?
motion detectors in home - is client moving about or is there an intruder?
communications - has client contacted anyone recently that may have been informed that client was not feeling well?
meal-times - has client eaten recently? Lack of food may indicate collapse due to hypoglycaemia in a client with diabetes mellitus and this information may be conveyed to the paramedics in the dispatched ambulance.

Once the controller has located the client, the system may activate an IP camera in the relevant room and start receiving video streams on the scene.

The invention includes a quality of life algorithm. The quality of life algorithm calculates a daily automatic moving average of "Quality of Life" (QoL) for each client. QoL is a gauge of the effectiveness of a system assisting an individual in their daily lives. A measurement of QoL is demonstrated by the system 1. The QoL index takes into account the variances of the exact definition of QoL, and measures the key domains that are generally regarded as contributing to QoL including general health and happiness, physical, emotional, cognitive and role functioning, social wellbeing and sexual functionality. It is flexible to allow any existing QoL algorithm that the caregiver has a preference for to be included within the overall QoL index. The QoL index is calculated based on the aggregation of scores from a number of separate tests measuring environmental parameters, activity levels, results from medical devices in the home and direct input questions from end user to "score" the quality of life. Data received from the monitoring devices 3 and data received from a user via the user interface of the monitoring hub 2 may be used to calculate the quality of life. The system 1 then compares the index to previous days and if it notices a pattern of decreasing QoL over a period of days it will trigger alerts into the control centre application when the quality of life is dropping. This is configurable for circumstances e.g. an individual with physical disabilities will not score lower on quality of life because they are not getting the same types of physical exercise. The data that is assessed may include the following, and the relative "weight" of each parameter is configurable:
- Physical Health:
   o Motion in the home
   o Time spent in bed
   o Time spent sitting down
   o Regularity of meals
   o Pedometer / Exercise / Physical activity
   o General pain levels - questionnaires - how are you feeling today?
- Psychological health:
   o Cognitive function - daily tests available to test this
   o General wellbeing - questionnaires - how are you feeling today?
- Environmental Factors
   o Home heating levels
   o Humidity levels
   o Weather / ability to leave home
- Social Relationships
   o Interactions with family and friends
   o Visitors to home by means of logbook on home user terminal
   o Communications

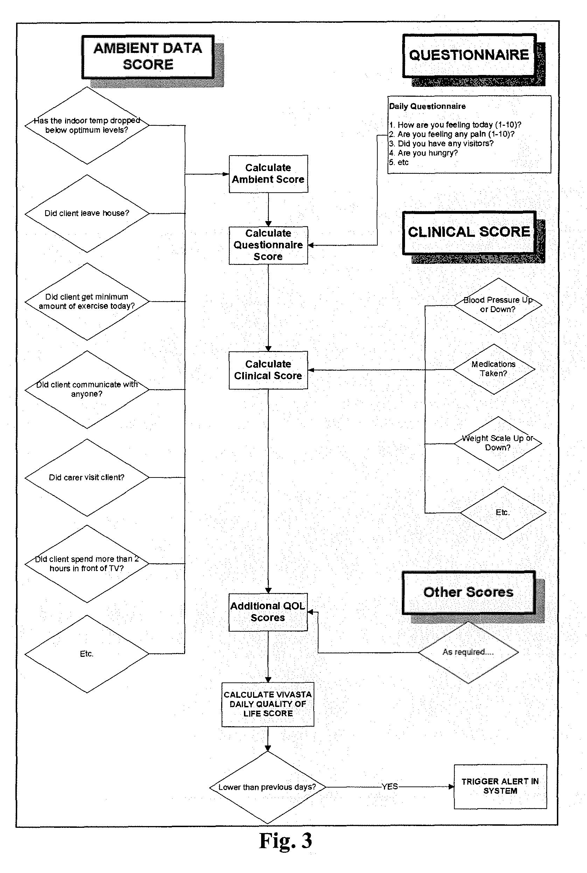

An example workflow for the quality of life calculations is shown in Fig. 3. All the criteria in the system are configurable and may be weighted differently for each individual.

The invention 1 collects a large amount of clinical data over time e.g. client health record including conditions, allergies, drug interactions, immunizations, test results, prescription medications, any diagnosed illnesses, any surgical procedures undertaken as well as a complete history of client vital observations over time such as weight, blood pressure and oxygen saturation. Clients may wish to export the data from the invention 1 into other platforms so that it can be reviewed by other healthcare professional outside of the invention 1. The system 1 integrates with online electronic health record systems which the client may authorise any healthcare professional or family or friend to view without giving them access to the invention 1. The upload of the full record of data captured by the invention 1 into these systems enables a thorough record of all actions to be recorded.

The system 1 has the capability to remotely configure the user interface to suit the needs and requirements of the individual. The system 1 has the capability to remotely configure the parameters and permutations of contextual data bundle gathered from different sensors 3 (health and care) depending on pre defined rules as prescribed based on requirements or needs or situation changes.

The system 1 has telemetry capabilities to monitor the status of all telecare and telehealth devices 3 and sensors 3 employed by the system 1 on site at a remote location, for example battery status, network connectivity and performance of computers. These may be configured remotely to suit individual requirements.

The system 1 enables third-parties to send their data through the system 1 without exposing their interfaces to the system 1. This means that third-parties may readily integrate their system into the invention 1 without exposing their information on the system 1. Third-party services include any living service such as home shopping, food delivery and home services and also medical monitoring which may be external to the system.

Third-party service providers such as taxi companies, home shopping services, medical monitoring services and mobile hairdressing may register their services automatically in the system 1. There is a comprehensive interface which may be used by third-parties which will automatically send new "order requests" to third-party systems. The third-party may then interface the order requests into their system using their own methods, without requiring extensive technical interaction. The invention 1 requires that all third-parties are approved before being added into the system 1, and thus helps to ensure that only pre-cleared service providers may offer services to clients. This may help protect the safety and security of clients.

In further detail, the invention 1 includes the following software components:

### Device call handler

This is an application which is connected through a modem or PBX system to a phone line and is used to receive and interpret calls from device hubs 3. The device call handler will answer the call and will send a "Data Request Tone" to the device hub 3 to start sending information. It may handle calls from device hubs 3 using different protocols such as BS 8521 2009. Once the device hub 3 has received a data request tone back, it will begin to send a stream of DTMF tones ("beeps") which indicate the identity of the device hub 3 and the type of telecare alert that has occurred. These tones are converted to DTMF characters (e.g. 1-9, A-D, * and #) and then sent to the RTC server 4.

### Home user application

This feature is designed to be used in the home on a touch-screen terminal or on a mobile device and includes the following functionality:
software panic connect button which may be used to initiate voice and video call to the controller;
communications functionality: enables voice calls, video calls, emails and SMS to family, friends, service providers, caregivers;
medication reminders: any missed medication reminders will be notified to the controller;
nutrition: detailed nutritional plans which may be confirmed by client;
third-party services ordering: e.g. shopping, ordering, taxi service or mobile hairdressing;
vital observations: e.g. weight, blood pressure may be entered manually or automatically through telehealth interface with devices in the home;
calendar: all events entered by controller or caregivers are visible here e.g. appointments, birthdays. Alerts for all events may be shown on this screen.

### Real-time communications servers

This feature is the central communications hub of the entire platform which performs the following functions:
enables all platform applications to create a continuous TCP/IP connection to this application. This application broadcasts application connectivity status to other relevant applications. SMS/email alerts may be generated if any key applications lose connectivity;
handles voice and video calls between client via home user application and controller via control centre application;
pushes all information received from client telecare sensors 3 which has been interpreted by device call handler application to the controller viewed in control centre application;
sends event notifications to all relevant applications when database is updated to prompt applications to resynchronise with the database server.

Database server application

This feature is the central repository of the system 1 containing all information including:
clients: age, address, location of client's home, contact details, photograph, medical information/record, prescribed medications, assigned caregivers and contact information, family and friends and contact information, any instructions specific to patient or to known patient complaints, details of any services ordered by patient, history of complaints, log of all calls including actions taken, history of patient vital observations, details of any medications and whether dosages have been missed, calendar of any events, appointments, assigned telecare and telehealth devices, subscribed third-party services etc;
controllers: login details, authorised clients;
machines: technical details of all registered machines e.g. home user terminals, control centre applications, servers;
caregivers: login details, location of home/workplace, speciality, assigned patients, contact information, schedule of availability.

When information is changed or new information is generated, this will be updated in the database. An event will be sent to the RTC server 4 to notify relevant applications to synchronise with the database. All synchronisations occur automatically and no user input is required.

### Control centre

This feature is designed to be monitored by a controller (person) and includes:
dashboard view of all registered clients in the system 1 and details of all clients as stored in the database;
action screen for responding to alerts:
   panic calls received from home user application panic connect button;
   device calls received from telecare system through device hub 3;
   rules-based alerts - system analysis of non-emergency data based on configurable rules within administration application such as front door sensor
   activated between 11pm-7am or weight gain over time;
actions may be initiated such as dispatching an ambulance which may be integrated with an ambulance dispatch system, sending an SMS which may be integrated with a mobile gateway, logging any advice given or contacting a third-party. All actions are logged in the database;
automatic responses may also be logged e.g. enabling SMS to be sent to family member automatically when alert occurs;
services screen for approving any requests for third-party services which have been requested on the home user terminal;
appointments screen for entering new appointments for any home users.

### Monitoring application including client monitoring services on each individual application

This feature provides a view of all machines used in the system 1 and their status e.g. servers, control centre applications, home user terminals etc. All applications may be accessed remotely for configuration and the system 1 includes technical information on every machine including performance and storage space. Information is sent to the monitoring application via a "Client Monitoring Service" which runs on each machine running any component of the invention.

### Family and friend portal and caregiver portal

This feature is a web-based application which allows authorised family and friends or caregivers to review details of the client such as:
any emergency alerts via telecare system or panic connect button;
any other actions that have occurred;
details of vital observations recorded;
details of any missed medication or event alerts;
details of any services ordered;
updating client calendar

### Administration application

This feature is used for configuration of all entities in the system such as:
creating new clients and updating information in the database;
creating new machines in the system 1 and assigning to users;
creating new caregivers in the system 1 including hospitals, specialities, assigning clients to caregivers, configuration of login details;
reporting including details of user concentrations, services usages, total machines types, patient subscribed services and warning of unassigned machines, patients without services, patients without contacts;
creation of rules for generating "Rules-based Alerts" in the control centre application. As well as panic calls and device calls, the system 1 allows alerts to be generated on data retrieved from non-emergency sensors in the home e.g. motion detectors, door sensors etc. These will not generate a device call unless the specific rule is recognised e.g. front door open between 11pm-7am.

### Third-party services integration

The system 1 may integrate with third-party services to provide additional services, such as home shopping or additional medical/healthcare monitoring through the home user terminal. A full third-party interface is available to enable third-parties to create DLLs that can be integrated into the system.

The integration between these components is shown in Fig. 1.

All data collected by the system 1 may be divided into two types of data - alarm data and historic data. The "Local Decision Machine" makes decisions on whether data is alarm data or the historical data analysis triggers an alert based on the "Rules-Based Alerts". The system 1 will send an alert to the control centre only if the data is alarm data. The system 1 is configurable to allow different criteria to be used for each individual on whether data is alarm data or historic data. For example, Client X may be configured:
- ALARM DATA
   o If pendant is pressed at any time
   o If front door is opened between 11.30pm-7am signal alarm
   o If bed pressure sensor is OFF (patient out of bed) between 11.30pm-7am AND bedroom door has been opened >15 mins previously and patient has not returned to bed
   o If smoke alarm is initiated at any time
   o If chair sensor in living room is ON for greater than 3 hours
- HISTORIC DATA
   o If motion detectors are activated
   o If any door sensors are opened (apart from rules above)
   o If any chair sensor is activated (apart from rules above)

The system 1 is configurable to allow different actions be taken on data and to determine when historic data will be uploaded to the database and therefore viewable within the control centre application, caregiver portal and family and friend portal. Some example possible configurations are shown below:
send all ALARM data and all HISTORIC data at all times;
send all ALARM data at all times and all HISTORIC data between hours of 5pm-9am;
send all ALARM data and store all HISTORIC data - do not send;
send all ALARM data and only send HISTORIC data for previous 30 minutes each time alarm occurs;
send all ALARM data and send HISTORIC data on request from control centre only.

All the aggregated data is available to the control centre which may request more data or make decisions on data as required. This means that all data is not sent regardless of circumstances and the controller may selectively choose which data to upload.

There are three types of alarms which are generated in the control centre application:
panic calls - home user software panic connect button;
device calls - telecare emergency devices e.g. pendant, smoke alarm;
rules-based alerts - based on combination of historical information from telecare and telehealth sensors.

Rules based alerts are based on an analysis of historic information by the local decision machine which is collected when the client:
performs any other action on home user application such as calling family member;
non-emergency devices are activated such as motion detectors, bed pressure sensors, fridge door sensors, general door sensors, chair pressure sensors;
vital observations are sent from medical devices such as blood pressure, weight, oxygen saturation level, pedometer.

Complex rules may be configured which will trigger alerts in the control centre similar to panic or device calls when specific combinations of information are generated. For example:
- Client X is at risk of forgetting to eat meals. Alert generated if:
   o Fridge door is not opened for more than 5 hours AND
   o Kitchen press door is not opened for more than 5 hours AND
   o Motion is detected around the home WHEN
   o Hours are between 7am-11pm
   o EVENT DATA: Send additional information on room client is currently in
- Client Y is at risk of wandering during the night. Alert generated if:
   o Front door is opened AND
   o Front gate is opened WHEN
   o Hours are between 11pm-6.30am
   o EVENT DATA: Send additional information on location of client
- Client Z is at risk of congestive heart failure, characterised by weight gain. Alert generated if:
   o Weight gain of more than 2 pounds on Day 1 AND
   o Weight gain of more than 2 pound on Day 2
   o OR
   o Weight gain of more than 5 pounds over 1 week

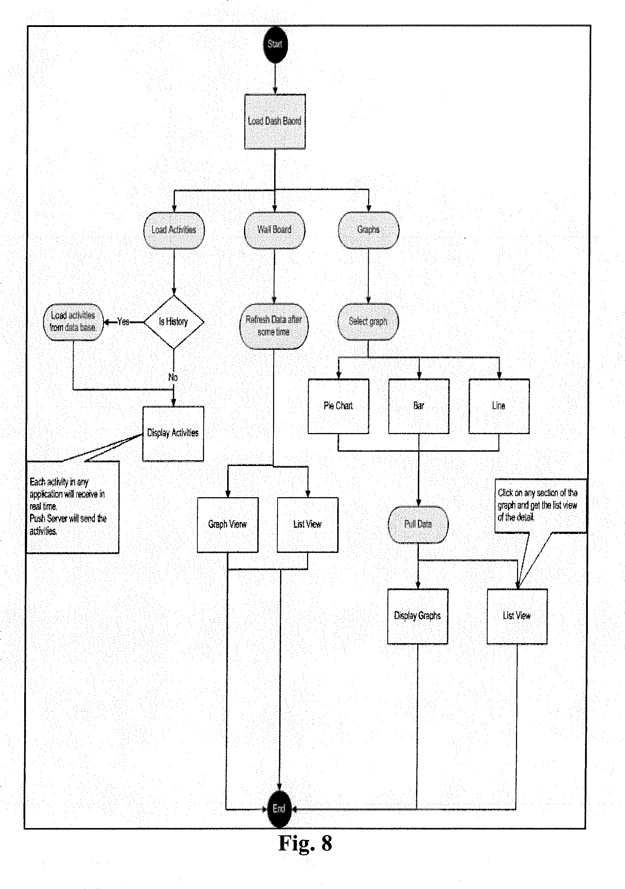

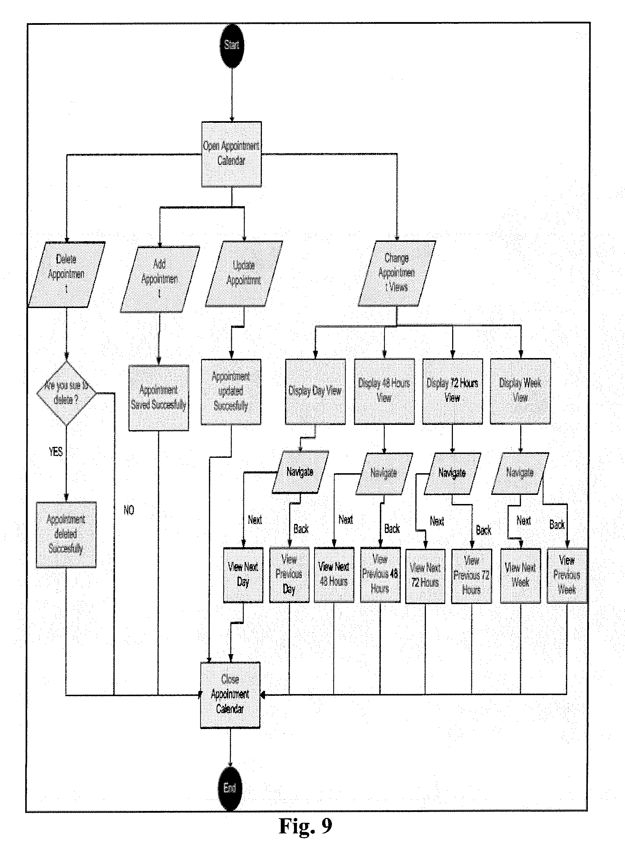

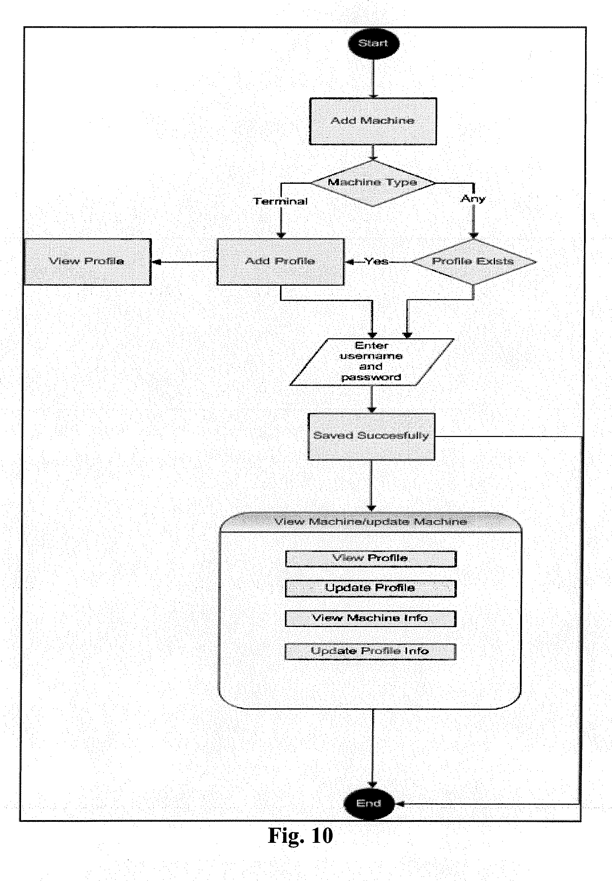

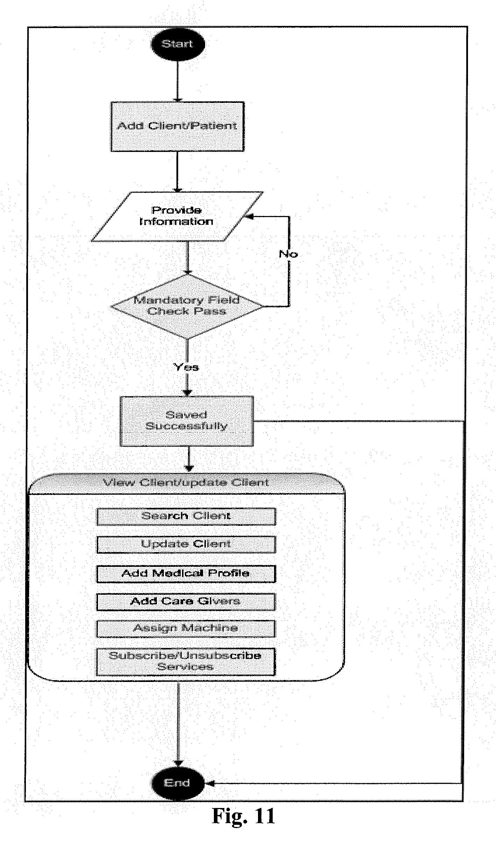

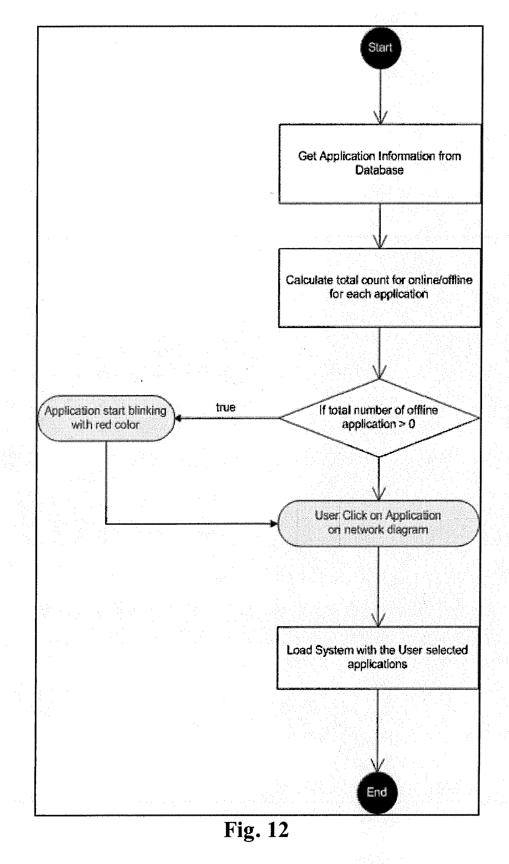

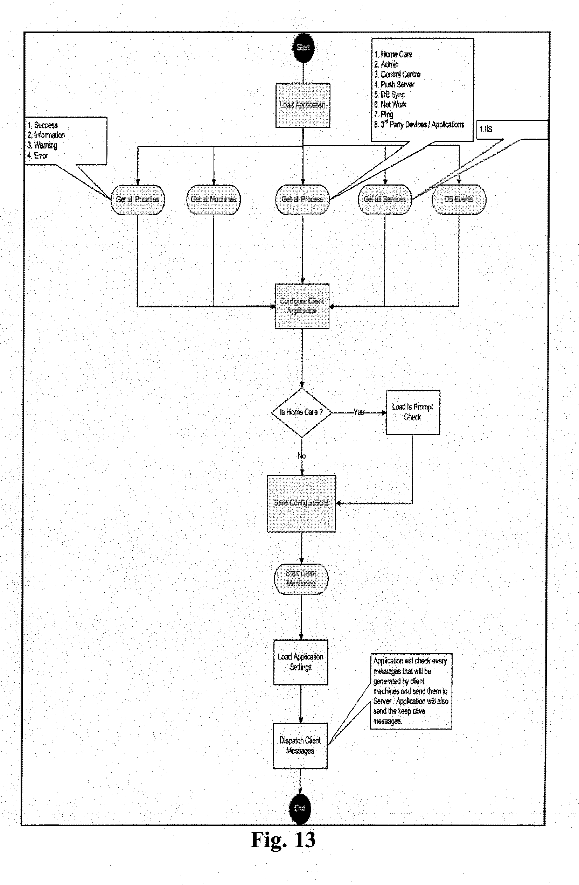

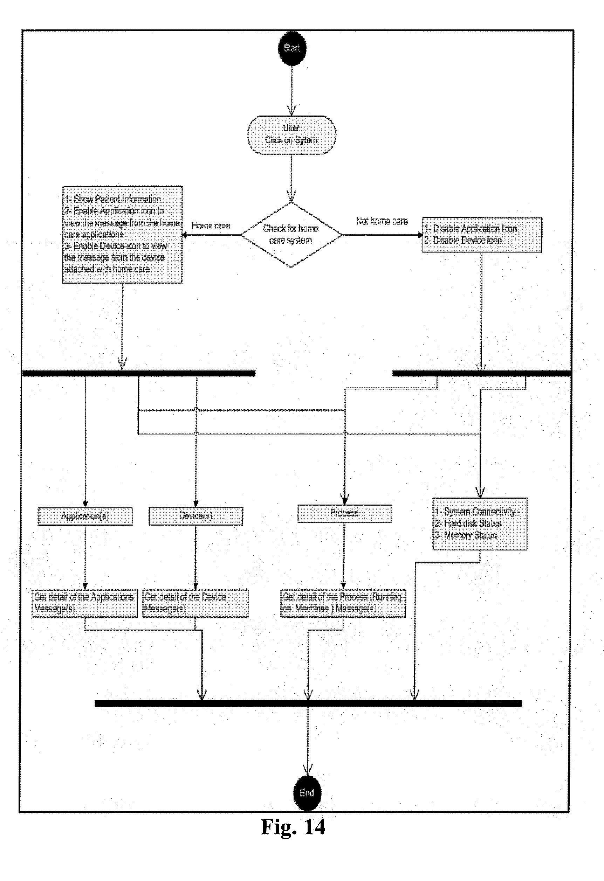

Fig. 8 shows the process flow of the control centre dashboard. This is the main screen that is presented to the controller and contains information on all clients in the system 1. Fig. 9 shows the process flow of creating appointments. Client appointments may be created in the control centre application and will be shown on the client home user screen. Fig. 10 shows the process flow of registering new administration machines. Fig. 11 shows the process flow of administration client registration. Fig. 12 shows the process flow of network monitoring. Fig. 13 shows the process flow of systems monitoring that is used to create an overview of all devices 3 online in the system 1. Fig. 14 shows the process flow of system detail summary in monitoring application.

In use, the monitoring hub 2 determines whether the monitoring hub 2 is connected in communication with the server 4 at the remote location, and determines the latency between the monitoring hub 2 and the server 4 at the remote location. The user interface of the monitoring hub 2 indicates to the user whether the monitoring hub 2 is connected in communication with the server 4 at the remote location, and indicates to the user the latency between the monitoring hub 2 and the server 4 at the remote location.

The monitoring hub 2 periodically transmits a plurality of communication protocol signals in series to each monitoring device 3 until communication is established with a particular monitoring device 3. The monitoring hub 2 may receive alarm data and/or non-alarm data from the plurality of monitoring devices 3. The monitoring hub 2 may receive alarm data and/or non-alarm data from a user via the user interface.

The monitoring hub 2 determines whether the data received is alarm data or non-alarm data. If the data received is determined to be alarm data, the monitoring hub 2 transfers the data received to the server 4 at the remote location. If the data received is determined to be non-alarm data, the monitoring hub 2 stores the data received locally at the monitoring hub 2.

The invention 1 provides an end-to-end managed care platform which enables services to be provided to individuals of all ages who require support in their daily living activities and healthcare. The invention 1 facilitates an end-to-end continuum of care service delivery within a single integrated platform. The invention 1 enables end-to-end data management. The system 1 facilitates complete end-to-end flow of data from multiple sources 3, as shown in Fig. 2.

Data is collected from both clinical and non-clinical data sensors 3 and devices 3 from multiple device manufacturers. Data is interfaced into the system 1 with multiple interfaces e.g. WiFi, bluetooth and other radio frequency, USB, ethernet, serial cable and any other interface. The data is available in multiple locations through caregiver portal, family and friend portal and admin application where it may be viewed by all authorised people including GP, hospital consultant, public health nurse.

The invention 1 facilitates remote monitoring, long-term condition management and a range of additional services within a single platform. Direct and extended care teams may proactively contribute to all aspects of an individual's care in an integrated manner, including co-ordination by dedicated monitoring centres.

The invention 1 combines environmental and clinical data within a single holistic approach to managing healthcare in the context of daily living activities of the individual in their ambient living environment, facilitating public and private care providers.

The invention 1 empowers individuals to become directly involved in their own care management throughout the course of their daily activities, regardless of location. The invention 1 provides a software platform that empowers older persons and those with long-term conditions to manage their health and wellbeing anytime, anywhere.

The embodiment of the invention described previously with reference to the accompanying drawings comprises a computer system and processes performed by the computer system. However the invention also extends to computer programs, particularly computer programs stored on or in a carrier adapted to bring the invention into practice. The program may be in the form of source code, object code, or a code intermediate source and object code, such as in partially compiled form or in any other form suitable for use in the implementation of the method according to the invention. The carrier may comprise a storage medium such as ROM, such as a CD-ROM, or magnetic recording medium, such as a floppy disk or hard disk. The carrier may be an electrical or optical signal which may be transmitted via an electrical or an optical cable or by radio or other means.

The invention is not limited to the embodiment hereinbefore described, with reference to the accompanying drawings, which may be varied in construction and detail.

## Claims

1. A care monitoring system comprising
means to receive alarm data and/or non-alarm data from one or more sources, means to determine whether the data received is alarm data, and
responsive to the data received being determined to be alarm data, means to transfer the data received to a remote location.

2. A system as claimed in claim 1 wherein the means to receive data is connectable in communication with one or more monitoring devices to receive alarm data and/or non-alarm data from the one or more monitoring devices.

3. A system as claimed in claim 2 wherein the means to receive data is connectable in communication with a first monitoring device by means of a first communication protocol, and the means to receive data is connectable in communication with a second monitoring device by means of a second communication protocol, the first communication protocol being different from the second communication protocol.

4. A system as claimed in claim 2 or 3 wherein the means to receive data is configured to establish communication with a monitoring device by transmitting a plurality of communication protocol signals to the monitoring device until communication is established.

5. A system as claimed in any of claims 1 to 4 wherein the means to receive data comprises a user interface to receive alarm data and/or non-alarm data from a user.

6. A system as claimed in any of claims 1 to 5 wherein responsive to the data received being determined to be non-alarm data, the system comprises means to store the data received locally.

7. A system as claimed in any of claims 1 to 6 wherein the system comprises means to determine whether the means to transfer the data is connected in communication with a remote location.

8. A system as claimed in claim 7 wherein the system comprises means to indicate to a user whether the means to transfer the data is connected in communication with a remote location.

9. A system as claimed in any of claims 1 to 8 wherein the system comprises means to determine the latency between the means to transfer the data and a remote location.

10. A system as claimed in claim 9 wherein the system comprises means to indicate to a user the latency between the means to transfer the data and a remote location.

11. A system as claimed in any of claims 1 to 10 wherein the means to transfer the data is configured to transfer the data by means of a telephone network.

12. A system as claimed in any of claims 1 to 11 wherein the means to transfer the data is configured to transfer the data by means of an internet connection.

13. A system as claimed in any of claims 1 to 12 wherein the system comprises one or more monitoring devices connected in communication with the means to receive data.

14. A method of care monitoring, the method comprising the steps of
receiving alarm data and/or non-alarm data from one or more sources, determining whether the data received is alarm data, and
responsive to the data received being determined to be alarm data, transferring the data received to a remote location.

15. A computer program product comprising computer program code capable of causing a computer system to perform a method as claimed in claim 14 when the computer program product is run on a computer system.
